# EUROPÄISCHE PATENTSCHRIFT

(11) **EP 2 262 788 B1**
(45) Veröffentlichungstag und Bekanntmachung des Hinweises auf die Patenterteilung: **24.02.2016**
(21) Anmeldenummer: 09714427.3
(22) Anmeldetag: 27.02.2009
(51) Int. Cl.: C07D 317/38

(54) **MEMBRANTRENNVERFAHREN ZUR HOCHSIEDERABTRENNUNG BEI DER HERSTELLUNG VON 1,3-DIOXOLAN-2-ONEN**
MEMBRANE SEPARATION METHOD FOR SEPARATING HIGH BOILER DURING THE PRODUCTION OF 1,3-DIOXOLANE-2-ONES
PROCÉDÉ DE SÉPARATION MEMBRANAIRE POUR SÉPARER LES PRODUITS À HAUT POINT D'ÉBULLITION LORS DE LA PRODUCTION DE 1,3-DIOXOLAN-2-ONES

(30) Priorität: 29.02.2008 EP 08152146
(43) Veröffentlichungstag der Anmeldung: 22.12.2010
(73) Patentinhaber: BASF SE, 67056 Ludwigshafen (DE)
(72) Erfinder: BIRNBACH, Stefan, 67246 Dirmstein (DE); KLINK, Hans, 67258 Heßheim (DE); MUGRAUER, Hans-Martin, 67112 Mutterstadt (DE); VOß, Hartwig, 67227 Frankenthal (DE)
(86) Internationale Anmeldenummer: PCT/EP2009/052354
(87) Internationale Veröffentlichungsnummer: WO 2009/106605

(56) Entgegenhaltungen:
- DE-A1- 19 819 586
- DATABASE CA [Online] CHEMICAL ABSTRACTS SERVICE, COLUMBUS, OHIO, US; CHOI, YOUNG CHUL: "Preparation method of alkylene carbonate of high purity by employing membrane separator" XP002530140 gefunden im STN Database accession no. 2007:811011 -& KR 2007 016 666 A (S. KOREA) 8. Februar 2007 (2007-02-08)

## Beschreibung

Die vorliegende Erfindung betrifft ein Verfahren zur kontinuierlichen Herstellung eines 1,3-Dioxolan-2-ons, bei dem man einen Austrag aus der Reaktionszone einer Auftrennung mittels einer semipermeablen Membran zur Abtrennung von polymeren Nebenprodukten unterzieht.

Es ist bekannt, 1,3-Dioxolan-2-one, wie Ethylencarbonat oder Propylencarbonat, durch Umsetzung eines entsprechenden Oxirans (wie Ethylenoxid oder Propylenoxid) mit Kohlendioxid in flüssiger Phase in Gegenwart eines in der flüssigen Phase homogen vorliegenden Katalysators herzustellen. Ein solches Verfahren ist z. B. in der DE 19819586 A1 beschrieben. Die Aufarbeitung des Reaktionsaustrags, d. h. die Isolierung des Produkts und die Abtrennung des Katalysators zur Rückführung in die Reaktionszone erfolgt dabei nach bekannten Verfahren, wie Destillation, Extraktion oder Strippen. Eine übliche Vorgehensweise umfasst die destillative Abtrennung von Niedrigsiedern und Produkt und die anschließende Rückführung des katalysatorhaltigen Sumpfprodukts in die Reaktion. Nachteilig an dieser Vorgehensweise ist, dass sich hochsiedende Nebenprodukte der Umsetzung, wie die aus den eingesetzten Oxiranen resultierenden cyclischen und linearen Polyether, im Reaktionssystem aufpegeln. Diese Hochsieder, die Molekulargewichte bis etwa 20000 Dalton aufweisen können, führen zu einer ansteigenden Viskosität im Katalysator-Rückführstrom. In regelmäßigen Abständen muss daher z. B. bei der destillativen Aufarbeitung der an Hochsiedern angereicherte Destillationssumpf gemeinsam mit dem darin enthaltenen Katalysator ausgeschleust werden. Dies führt zu einer wirtschaftlichen Benachteiligung des Verfahrens durch den erforderlichen Stillstand der Anlage sowie den bei der Hochsiederentsorgung anfallenden Katalysator- und Produktverlust. Es besteht daher Bedarf an einem Verfahren zur kontinuierlichen Herstellung von 1,3-Dioxolan-2-onen, das diese Nachteile nicht aufweist.

Es ist bekannt, zur Abtrennung von homogenen Katalysatoren bei kontinuierlichen Syntheseverfahren semipermeable Membranen einzusetzen. Derartige Verfahren sind z. B. in der DE 10328713 A1 und DE 10328715 A1 beschrieben. Diesen Verfahren liegt jedoch eine chemisch andere Problemstellung zu Grunde, nämlich die Addition zweier terminaler Olefine, die mindestens zwei funktionelle Gruppen tragen. Bei der Aufarbeitung des Reaktionsaustrags der Herstellung von 1,3-Dioxolan-2-onen fallen die Hochsieder im Gemisch mit den in der Regel auch als Reaktionslösungsmittel eingesetzten 1,3-Dioxolan-2-onen und dem Katalysator an. Da die Bildung von 1,3-Dioxolan-2-onen im Gegensatz zur in der DE 10328713 A1 und DE 10328715 A1 beschrieben Chemie eine Gleichgewichtsreaktion ist, ist in diesem Fall mit einer irreversiblen Verblockung der Membran, ausgelöst durch das bei der Rückreaktion gebildete Oxiran (z. B. durch Reaktion des Oxirans mit reaktiven funktionellen Gruppen auf der Oberfläche und/oder in den Poren der Membran) oder durch die Ablagerung polymerer Produkte des Oxirans auf und in der Membran zu rechnen. Dabei war weiterhin zu erwarten, dass die Kettenverlängerung der Polymere, die sich auf der Oberfläche und/oder in den Poren der Membran abgelagert haben, durch weitere Addition von Oxiran eine negative Rolle bezüglich einer irreversiblen Verblockung der Membran spielen.

KR 2007 016 666 beschreibt ein kontinuierliches katalytisches Verfahren zur Herstellung von Alkylencarbonaten aus Alkylenoxiden und CO2 und Auftrennung des Produktgemisches mittels Membrantrennverfahren.

DE 198 19 586 beschreibt ein kontinuierliches katalytisches Verfahren zur Herstellung von 1,3-Dioxolan-2-onen aus CO2 und Oxiranen ohne Membrantrennschritt.

Überraschenderweise wurde nun gefunden, dass es möglich ist, aus dem Reaktionsaustrag der 1,3-Dioxolan-2-on-Herstellung die hochsiedenden (polymeren) Nebenprodukte durch ein Membrantrennverfahren abzutrennen. Dabei ist es insbesondere überraschend, dass die Abtrennung der Hochsieder aus dem Retentat der Membrantrennung möglich ist, ohne dass es zu einer merklichen Verringerung der Durchlässigkeit der Membran durch Ablagerung oder Aufbau von Polymeren auf der Membranoberfläche und/oder in den Membranporen kommt. Das erfindungsgemäße Verfahren eignet sich somit in vorteilhafter Weise auch zur kontinuierlichen Abtrennung der gebildeten polymeren Nebenprodukte. Sowohl ein regelmäßiger Stillstand der Anlage als auch ein unwirtschaftlicher Katalysatorverlust können vermieden werden.

Gegenstand der Erfindung ist daher ein Verfahren zur kontinuierlichen Herstellung eines 1,3-Dioxolan-2-ons der allgemeinen Formel I 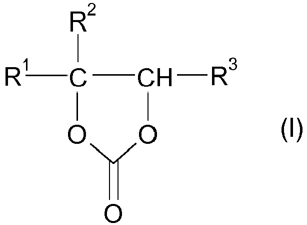 worin
R¹ für Wasserstoff oder einen organischen Rest mit 1 bis 40 Kohlenstoffatomen steht,
R² und R³ unabhängig voneinander für Wasserstoff oder C₁-C₄-Alkyl stehen, wobei R² und R³ auch miteinander zu einem fünf- oder sechsgliedrigen Ring verbunden sein können, bei dem man:
   a) in einer Reaktionszone ein Oxiran der allgemeinen Formel II 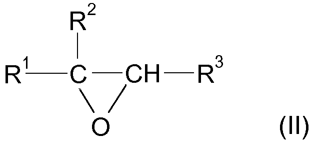 worin R¹, R² und R³ die zuvor angegebenen Bedeutungen besitzen, mit Kohlendioxid in flüssiger Phase in Gegenwart eines in der flüssigen Phase homogen vorliegenden Katalysators umsetzt,
   b) aus der Reaktionszone einen flüssigen Austrag entnimmt, der polymere Nebenprodukte der Umsetzung enthält, und einer Aufarbeitung unterzieht, die eine Auftrennung mittels einer semipermeablen Membran unter Erhalt eines Permeats und eines Retentats umfasst, wobei ein hochmolekularer Anteil der polymeren Nebenprodukt von der Membran zurückgehalten wird, wobei in Schritt b) vom Austrag aus der Reaktionszone ein im Wesentlichen aus der Verbindung (I), dem Katalysator und den polymeren Nebenprodukten bestehenden Strom abgetrennt und zumindest teilweise der Auftrennung mittels einer semipermeablen Membran unterzogen wird, wobei der Katalysator zumindest teilweise in das Permeat übergeht, wobei der Permeat wenigstens 70 % des in dem zur Membrantrennung eingesetzten Strom enthaltenen Katalysators enthält, und
   c) aus dem Retentat einen den hochmolekularen Anteil der polymeren Nebenprodukte enthaltenden Ausschleusstrom bereitstellt.

Die Herstellung der Verbindungen (I) führt zur Bildung von hochsiedenden Nebenprodukten, wobei es sich im Wesentlichen um Oligomere und Polymere handelt, die sich von den zur Reaktion eingesetzten Oxiranen (II) ableiten. Dabei kann es sich sowohl um lineare als auch cyclische Verbindungen handeln. Diese Nebenprodukte werden im Rahmen der vorliegenden Erfindung unter dem Begriff "polymere Nebenprodukte" zusammengefasst.

Bedingt durch die kontinuierliche Reaktionsführung kommt es zu Beginn des erfindungsgemäßen Verfahrens zunächst zu einer Zunahme des Molekulargewichts der im Austrag aus der Reaktionszone enthaltenen polymeren Nebenprodukte, bis die Trenngrenze der eingesetzten Membran(en) erreicht ist. Danach stellt sich bedingt durch die Ausschleusung des hochmolekularen Anteils im Wesentlichen ein stationärer Zustand ein, d. h. die Konzentration an polymeren Nebenprodukten im Austrag aus der Reaktionszone steigt im Wesentlichen nicht mehr an. Der "hochmolekularer Anteil" der polymeren Nebenprodukte ist dabei im Rahmen der Erfindung derjenige Anteil, der durch die Membran zurückgehalten wird. Der "niedermolekularer Anteil" der polymeren Nebenprodukte ist entsprechend derjenige Anteil, der befähigt ist, durch die Membran in das Permeat überzutreten (d. h. die Trenngrenze der eingesetzten Membran bestimmt was im Sinne der Erfindung der niedermolekulare und was der hochmolekulare Anteil der polymeren Nebenprodukte ist). Aus dem mit dem Katalysator in die Reaktion zurückgeführten niedermolekularen Anteil bilden sich durch weitere Addition von Oxiran wieder höhermolekulare Nebenprodukte.

Zur Auftrennung mittels einer semipermeablen Membran in Schritt b) wird ein Strom eingesetzt, der im Wesentlichen aus dem 1,3-Dioxolan-2-ons der allgemeinen Formel I (Produkt), dem Katalysator und den polymeren Nebenprodukten besteht. Der Katalysator und das Produkt gehen dabei zumindest teilweise in das Permeat über. Bevorzugt ist ein Verfahren, bei dem man zusätzlich:
d) das Permeat zumindest teilweise in die Reaktionszone in Schritt a) und/oder die Aufarbeitung in Schritt b) zurückführt.

Die Umsetzung des Oxirans (II) mit Kohlendioxid in Schritt a) erfolgt in einer Reaktionszone, die einen oder mehrere (z. B. zwei, drei oder mehr als drei) Reaktoren aufweisen kann. Bei den Reaktoren kann es sich um gleiche oder verschiedene Reaktoren handeln. Diese können z. B. jeweils gleiche oder verschiedene Vermischungscharakteristiken aufweisen und/oder durch Einbauten ein- oder mehrfach unterteilt sein. Geeignete druckfeste Reaktoren zur Herstellung der 1,3-Dioxolan-2-one der Formel I sind dem Fachmann bekannt. Dazu zählen die allgemein üblichen Reaktoren für Gas-Flüssig-Reaktionen, wie z. B. Rohrreaktoren, Rohrbündelreaktoren, Gasumlaufreaktoren, Blasensäulen, Schlaufenapparate, Rührkessel (die auch als Rührkesselkaskaden ausgestaltet sein können), Air-Lift-Reaktoren etc.

Ein geeignetes Verfahren zur Umsetzung in einem zweiteiligen Reaktor, bei dem im ersten Teil die Umsetzung unter Rückvermischung bis zu einem Umsatz des Oxirans von mindestens 80 % und im zweiten Teil die Umsetzung unter nicht rückvermischenden Bedingungen erfolgt, wobei das Kohlendioxid in der gesamten Reaktionszone im Gegenstrom zum Oxiran geführt wird, ist in der DE 19819586 beschrieben.

Die Temperatur bei der Umsetzung in Schritt a) beträgt im Allgemeinen etwa 60 bis 160 °C, vorzugsweise 70 bis 150 °C, besonders bevorzugt 90 bis 145 °C. Bei einer Durchführung der Reaktion in mehr als einem Reaktor kann in jedem nachgeschalteten Reaktor eine andere Temperatur eingestellt werden als im vorhergehenden Reaktor. In einer speziellen Ausführungsform wird der jeweils nachgeschaltete Reaktor mit einer höheren Temperatur betrieben als der vorhergehende Reaktor. Zusätzlich kann jeder Reaktor zwei oder mehrere Reaktionszonen aufweisen, die bei unterschiedlicher Temperatur betrieben werden. So kann beispielsweise in einer zweiten Reaktionszone eine andere, vorzugsweise eine höhere Temperatur als in der ersten Reaktionszone bzw. in jeder nachfolgenden Reaktionszone eine höhere Temperatur als in einer vorhergehenden Reaktionszone eingestellt werden, z. B. um einen möglichst vollständigen Umsatz zu erzielen.

Der Reaktionsdruck in Schritt a) beträgt im Allgemeinen etwa 2 bis 50 bar, besonders bevorzugt 5 bis 40 bar, insbesondere 10 bis 30 bar. Gewünschtenfalls kann beim Einsatz mehrerer Reaktoren in jedem nachfolgenden Reaktor ein anderer (vorzugsweise höherer) Druck eingestellt werden als im vorherigen Reaktor.

Die Einsatzstoffe Kohlendioxid und Oxiran können in der Reaktionszone im Gleichstrom oder im Gegenstrom geführt werden. Möglich ist auch eine Ausführungsform, bei der Kohlendioxid und Oxiran in einem Teil der Reaktionszone im Gleichstrom und in einem anderen Teil im Gegenstrom geführt werden. Bevorzugt werden in der gesamten Reaktionszone Kohlendioxid und Oxiran im Gegenstrom geführt.

Aus der Reaktionszone wird erfindungsgemäß ein flüssiger Austrag entnommen und für die folgende Aufarbeitung eingesetzt. Zusätzlich kann am Kopf des Reaktors oder, bei einer Reaktionszone aus mehreren Reaktoren, eines der Reaktoren, ein gasförmiger Austrag entnommen werden. Dieser enthält nicht umgesetztes Kohlendioxid, sowie gegebenenfalls weitere gasförmige Bestandteile, wie Oxiran (II) und/oder Inerte (Edelgase, Stickstoff). Der gasförmige Austrag kann gewünschtenfalls teilweise oder vollständig wieder der Reaktionszone zugeführt werden. Gewünschtenfalls kann der gasförmige Austrag auch teilweise oder vollständig ausgeschleust werden, um ein Aufpegeln von inerten gasförmigen Bestandteilen in der Reaktionszone zu vermeiden.

Als Katalysatoren für das erfindungsgemäße Verfahren können aus der Literatur, z. B. aus US-A 2773070, US-A 2773881, Chem. Lett. (1979) S. 1261, Chem. Lett. (1977) S. 517, DE-A 3529263, DE-B 1169459, EP-A 069494 oder EP-B 543249, für derartige Umsetzungen bekannte Katalysatoren eingesetzt werden. Vorzugsweise setzt man als Katalysatoren Oniumsalze oder Metallsalze oder Mischungen hieraus ein.

Als Oniumsalze eignen sich prinzipiell alle Verbindungen dieses Typs, darunter besonders Ammonium-, Phosphonium- und Sulfoniumsalze der allgemeinen Formel IIIa bis IIIc 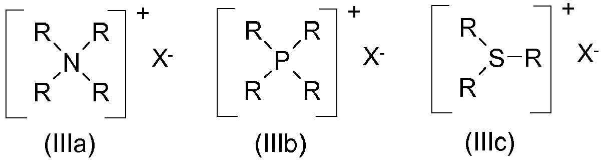 in denen die Substituenten R gleiche oder verschiedene Kohlenwasserstoffrest mit jeweils 1 bis 20 C-Atomen bedeuten, wobei die Summe der C-Atome in den Resten R jeweils nicht größer als 24 ist, und in denen X- für ein Anionäquivalent, vorzugsweise Halogenid, insbesondere Bromid oder Iodid, steht.

Bevorzugt sind Ammoniumsalze der Formel IIIa, insbesondere Tetraethylammoniumbromid. Daneben sind diejenigen Verbindungen IIIa bevorzugt, in denen drei der Reste R für C₁-C₄-Alkylgruppen, wie Methyl oder Ethyl, stehen und der vierte Rest R für Benzyl oder unverzweigtes C₆- bis C₁₈-Alkyl steht.

Bevorzugt als Katalysator sind weiterhin Phosphoniumsalzen IIIb, die sich vom Triphenylphosphin ableiten und deren vierter Substituent durch Quaternierung mit einem C₁ bis C₆-Alkylbromid in das Molekül eingeführt wurde.

Ein geeignetes Sulfoniumsalz IIIc ist beispielsweise das leicht herstellbare Trimethylsulfoniumiodid. Im Allgemeinen sind die Ammonium- und Phosphoniumsalze besser geeignet als die Sulfoniumsalze.

Allgemein können die Kohlenwasserstoffreste R in den Verbindungen IIIa bis IIIc verzweigte oder vorzugsweise unverzweigte C₁- bis C₂₀-Alkylgruppen, Arylalkylgruppen wie die Benzylgruppen, die Cyclohexylgruppe und aromatische Gruppen wie die Phenyl- oder die p-Tolylgruppe bedeuten. Ferner können Alkylreste R auch miteinander verbunden sein, etwa unter Ausbildung eines Piperidinrings. Als Anionen kommen außer Halogenid beispielsweise Sulfat und Nitrat in Betracht.

Häufig, und zwar besonders im Falle der Oniumbromide, ist es nicht erforderlich, von den Salzen IIIa bis IIIc selber auszugehen, sondern es genügt, deren Vorprodukte, Base und Quaternierungsreagens einzusetzen, aus denen sich die wirksamen Quaternierungsprodukte IIIa bis IIIc in situ bilden.

Als Metallsalze kommen Salze von Alkalimetallen, Erdalkalimetallen und Übergangsmetallen, insbesondere von zweiwertigen Übergangsmetallen, in Betracht, beispielsweise Natrium-, Kalium-, Magnesium-, Calcium-, Aluminium-, Mangan(II)-, Eisen(II)-, Nickel(II)-, Kupfer(II)-, Zink, Cadmium- oder Blei(II)-Salze. Als Anionen für diese Salze eignen sich Sulfat, Nitrat, Phosphat, Carbonat, Acetat, Formiat sowie vor allem Halogenide wie Chlorid, Bromid und Iodid. Besonders gute Resultate erzielt man mit Zinksalzen wie Zinksulfat, Zinknitrat, Zinkphosphat, Zinkcarbonat, Zinkacetat, Zinkformiat, Zinkchlorid, Zinkbromid oder Zinkiodid. Man kann selbstverständlich auch Mischungen derartiger Metallsalze einsetzen, das gleiche gilt auch für die oben genannten Oniumsalze. Auch Mischungen von Oniumsalzen mit Metallsalzen sind möglich und zeigen in einigen Fällen überraschende Vorteile.

Die Menge der als Katalysatoren eingesetzten Oniumsalze und/oder der Metallsalze ist in der Regel unkritisch. Bevorzugt werden etwa 0,01 bis 3 Gew.-%, bezogen auf eingesetztes Oxiran (II), verwendet.

In einer bevorzugten Ausführungsform setzt man als Katalysatoren Alkalimetallbromide, Alkalimetalliodide, Tetraalkylammoniumbromide, Tetraalkylammoniumiodide, Halogenide zweiwertiger Metalle oder Mischungen hieraus ein.

In einer ganz besonders bevorzugten Ausführungsform setzt man als Katalysatoren eine Mischung aus Oniumsalzen, insbesondere Ammonium-, Phosphonium- und/oder Sulfoniumsalzen der allgemeinen Formel IIIa bis IIIc, und Zinksalzen, insbesondere den oben explizit genannten, ein. Die wirksamen Mengen der Zinksalze liegen hierbei je nach der Reaktivität des eingesetzten Oxirans, der Aktivität des Oniumsalzes und den sonstigen Reaktionsbedingungen zwischen 0,1 bis 1,0 mol, vorzugsweise zwischen 0,3 und 0,7 mol, pro Mol des Oniumsalzes.

Für das erfindungsgemäße Verfahren geeignete inerte Lösungsmittel sind z. B. Dioxan, Toluol oder Aceton. Wird zur Umsetzung ein Lösungsmittel eingesetzt, so normalerweise in Mengen von etwa 10 bis 100 Gew.-%, bezogen auf das eingesetzte Oxiran (II). Ist das Verfahrensprodukt I unter den Reaktionsbedingungen flüssig, verwendet man zweckmäßigerweise dieses als Lösungsmittel, vorzugsweise als alleiniges Lösungsmittel. Es hat sich in solchen Fällen als günstig erwiesen, den Katalysator in dem Verfahrensprodukt zu lösen und diese Lösung zuzudosieren, wobei praktisch keine weiteren Lösungsmittel im Umsetzungsreaktor zugegeben sind. Hierbei beträgt die Konzentration des Katalysators im Verfahrensprodukt (I)I üblicherweise 0,5 bis 20 Gew.-%, insbesondere 1 bis 15 Gew.-%. Das molare Verhältnis von in der gleichen Zeiteinheit zugegebener Eduktmenge (II) zu mit dem Katalysator zugegebenem Verfahrensprodukt (I) liegt in der Regel bei 100:1 bis 1:1, insbesondere 50:1 bis 2:1.

Beim erfindungsgemäßen Verfahren setzt man die Eduktströme an Oxiran (II) und Kohlendioxid vorzugsweise im molaren Verhältnis von 1:1 bis 1:1,05, insbesondere 1:1 bis 1:1,02 ein. Ein möglicher leichter Überschuss an Kohlendioxid ist vorteilhaft, um die Verluste an Kohlendioxid beim Entspannen des Austrags aus der Reaktionszone auszugleichen.

Normalerweise erzielt man mit dem erfindungsgemäßen Verfahren praktisch quantitative Umsätze an (II), in der Regel mindestens 99 %, insbesondere mindestens 99,5 %, vor allem mindestens 99,9 %.

Geeignete Reste R¹ sind:
- Wasserstoff,
- gesättigte und ungesättigte verzweigte und unverzweigte aliphatische Reste mit bis zu 40 C-Atomen, insbesondere bis zu 18 C-Atomen,
- iso- oder heterocyclische cycloaliphatische Gruppen mit vorzugsweise 5 bis 7 Ringgliedern,
- iso- oder heterocyclische aromatische Gruppen und
- gemischte Reste mit Gruppierungen der vorgenannten Art, beispielsweise araliphatische Reste wie die Benzylgruppe.

Die von Wasserstoff verschiedenen Reste R¹ können einen oder mehrere Substituenten wie Halogen, Nitrogruppen, freie oder substituierte Aminogruppen, Hydroxylgruppen, Formylgruppen oder Cyangruppen tragen oder Ether-, Keton- oder Estergruppierungen enthalten. Bevorzugt steht R¹ für Wasserstoff.

Bei den Resten R² und R³ handelt es sich in der Regel um Wasserstoff oder die Methylgruppe sowie um solche Reste, die miteinander zu einem fünf- oder sechsgliedrigen Ring verbunden sind, wofür Cyclohexenoxid als Verbindung II ein Beispiel ist. Enthält II zwei Oxiranringe mit jeweils einer (CH₂-)Gruppierung, so enthält man die entsprechenden Bis-Dioxolane I; Oxiranringe, die an beiden C-Atomen substituiert sind, werden in der Regel langsamer angegriffen als solche, die nur an einem der C-Atome substituiert sind. Bevorzugt wird als Oxiran (II) Ethylenoxid oder Propylenoxid, speziell Ethylenoxid, eingesetzt.

In einer bevorzugten Ausführungsform stellt man mit dem erfindungsgemäßen Verfahren Ethylencarbonat oder Propylencarbonat her.

Aus der Reaktionszone wird erfindungsgemäß ein flüssiger Strom als Austrag entnommen und einer Aufarbeitung unterzogen (= Schritt b) des erfindungsgemäßen Verfahrens).

Der aus der Reaktionszone entnommene flüssige Austrag enthält im Allgemeinen die folgenden Bestandteile:
- 1,3-Dioxolan-2-on der Formel I,
- Katalysator,
- polymere Nebenprodukte,
- gegebenenfalls zur Reaktion eingesetztes Lösungsmittel,
- gegebenenfalls gelöstes Kohlendioxid,
- gegebenenfalls gelöstes Oxiran der Formel II.

Die Aufarbeitung in Schritt b) umfasst als einen wesentlichen Schritt ein Membrantrennverfahren. Dabei gelingt es in vorteilhafter Weise, den zur Reaktion eingesetzten Katalysator und die bei der Reaktion gebildeten Hochsieder soweit zu trennen, dass eine katalysatorarme oder im Idealfall katalysatorfreie Hochsiederausschleusung ermöglicht wird.

Erfindungsgemäß enthält das Permeat (bei einer mehrstufigen Membrantrennung bezogen auf alle Stufen) wenigstens 70 Gew.-%, bevorzugt wenigstens 80 Gew.-%, insbesondere wenigstens 90 Gew.-%, des in dem zur Membrantrennung eingesetzten Strom enthaltenen Katalysators.

Vorzugsweise enthält der Austrag aus der Reaktionszone einen Anteil an polymeren Nebenprodukten von höchstens 6 Gew.-%, besonders bevorzugt höchstens 5 Gew.-%, insbesondere höchstens 4 Gew.-%, bezogen auf das Gesamtgewicht des Reaktionsaustrags.

Vorzugsweise wird zur Membrantrennung in Schritt b) nicht unmittelbar der flüssige Austrag aus der Reaktionszone eingesetzt, sondern dieser zunächst einer Abtrennung eines Teils der darin enthaltenen Komponenten unterzogen. Erfindungsgemäß wird in Schritt b) vom Austrag aus der Reaktionszone ein im Wesentlichen aus der Verbindung (I), dem Katalysator und den polymeren Nebenprodukte bestehender Strom abgetrennt. Erfindungsgemäß wird dieser Strom dann zumindest teilweise der Auftrennung mittels einer semipermeablen Membran unterzogen. In einer speziellen Ausführungsform teilt man diesen Strom in einen ersten und einen zweiten Teilstrom, wobei der erste Teilstrom in die Reaktionszone zurückgeführt und der zweite Teilstrom der Auftrennung mittels einer semipermeablen Membran unterzogen wird.

Bevorzugt wird vom Austrag aus der Reaktionszone vor der Membrantrennung darin gelöstes Kohlendioxid und/oder Oxiran der Formel II zumindest teilweise abgetrennt.

Des Weiteren bevorzugt wird vom Austrag aus der Reaktionszone vor der Membrantrennung ein Strom abgetrennt, der im Wesentlichen aus dem Reaktionsprodukt, d. h. der Verbindung (I) besteht.

Wie zuvor ausgeführt, kann die Abtrennung von Kohlendioxid und/oder Oxiran über einen separaten gasförmigen Austrag aus der Reaktionszone erfolgen. Ist das erfindungsgemäße Verfahren als reines Flüssigaustragsverfahren ausgestaltet, so kann der Austrag aus der Reaktionszone zur Abtrennung des darin gelösten Kohlendioxids und/oder Oxirans (II) zuerst einem Entspannungsschritt unterzogen werden. Dabei erfolgt dann in der Regel eine Auftrennung in eine Flüssigphase, die im Wesentlichen aus der Verbindung (I), polymeren Nebenprodukten, dem homogen gelösten Katalysator und gegebenenfalls geringen Mengen an gelöstem Kohlendioxid und/oder Oxiran (II) besteht, und eine Gasphase, die im Wesentlichen aus Kohlendioxid und/oder Oxiran (II) besteht. Die in dem Entspannungsschritt resultierende Gasphase kann teilweise oder vollständig in die Reaktionszone zurückgeführt werden. Diese Rückführung kann gemeinsam mit einem der in die Reaktionszone eingespeisten Gasströme oder separat erfolgen. Die in dem Entspannungsschritt erhaltene Flüssigphase wird vorzugsweise einer weiteren Auftrennung nach einem üblichen, dem Fachmann bekannten Verfahren unterzogen. Bevorzugt wird die Flüssigphase einer Destillation unter Erhalt eines im Wesentlichen aus der Verbindung (I) bestehenden Stroms und eines im Wesentlichen aus der Verbindung (I), dem Katalysator und den polymeren Nebenprodukten bestehenden Stroms unterzogen. Der letztgenannte Strom kann dann zur Membrantrennung eingesetzt werden.

Vorzugsweise enthält dieser Strom einen Anteil an hochsiedenden Nebenprodukten von höchstens 30 Gew.-%, bevorzugt höchstens 25 Gew.-%, besonders bevorzugt höchstens 20 Gew.-%, bezogen auf das Gesamtgewicht des im Wesentlichen aus der Verbindung (I), dem Katalysator und den polymeren Nebenprodukten bestehenden Stroms.

In einer speziellen Ausführungsform wird der letztgenannte Strom in einen ersten und einen zweiten Teilstrom getrennt, wobei der erste Teilstrom in die Reaktionszone zurückgeführt und der zweite Teilstrom zur Membrantrennung eingesetzt wird.

Alternativ kann man den Austrag aus der Reaktionszone unmittelbar einer destillativen Auftrennung in
- einen im Wesentlichen aus nicht umgesetztem Kohlendioxid und/oder Oxiran (II) bestehenden Strom,
- einen im Wesentlichen aus der Verbindung (I) bestehenden Strom, und
- einen im Wesentlichen aus der Verbindung (I), dem Katalysator und den polymeren Nebenprodukten bestehenden Strom unterziehen.

Die destillative Auftrennung des Reaktionsaustrags kann nach üblichen, dem Fachmann bekannten Verfahren erfolgen. Geeignete Vorrichtungen für die destillative Auftrennung umfassen Destillationskolonnen, wie Bodenkolonnen, die mit Glocken, Siebplatten, Siebböden, Packungen, Einbauten, Ventilen, Seitenabzügen etc. versehen sein können. Geeignet sind speziell Trennwandkolonnen, die mit Seitenabzügen, Rückführungen etc. versehen sein können. Zur Destillation kann eine Kombination aus zwei oder mehr als zwei Destillationskolonnen eingesetzt werden. Geeignet sind weiterhin Verdampfer, wie Dünnschichtverdampfer, Fallfilmverdampfer, Sambay-Verdampfer etc. und Kombinationen davon.

Die Destillation erfolgt vorzugsweise bei einer Sumpftemperatur im Bereich von etwa 30 bis 160 °C, besonders bevorzugt 50 bis 150 °C, insbesondere 70 bis 140 °C.

Die Destillation kann unter Normaldruck, erhöhtem Druck oder vermindertem Druck durchgeführt werden. Vorzugsweise liegt der Druck bei der Destillation in einem Bereich von etwa 0,0005 bar bis 1,5 bar, besonders bevorzugt 0,001 bar bis 1,2 bar, insbesondere 0,01 bar bis 1,1 bar.

Zur Abtrennung von polymeren Nebenprodukten wird ein aus dem Austrag der Reaktionszone erhältlicher Strom, der zusätzlich eine Verbindung (I) und den Katalysator enthält, unter Druck mit einer Membran in Kontakt gebracht und ein den niedermolekularen Anteil der polymeren Nebenprodukte und den gelösten Katalysator enthaltendes Permeat (Filtrat) auf der Rückseite der Membran bei einem geringeren Druck als auf der Feedseite abgezogen. Als Retentat erhält man eine aufkonzentrierte Lösung des hochmolekularen Anteils der polymeren Nebenprodukte (hochsiedenden Verunreinigungen), die an Katalysator abgereichert ist.

In einer bevorzugten Ausführung erfolgt die Auftrennung mittels Membran in Schritt b) in zwei oder mehr als zwei Stufen (z. B. in 3, 4, 5 oder 6).

In einer bevorzugten Ausführungsform wird die bei der Membranauftrennung abgetrennte Menge an Permeat zumindest teilweise im Retentat durch Flüssigkeitszugabe ergänzt. Diese Ergänzung kann kontinuierlich oder diskontinuierlich erfolgen. Eine Membrantrennung (Ultrafiltration), bei der der zurückgehaltene Stoff nicht aufkonzentriert wird, sondern bei der die abgetrennte Menge an Permeat ergänzt wird, wird auch als Diafiltration bezeichnet. Wenn die Auftrennung mittels Membran in Schritt b) in zwei oder mehr als zwei Stufen erfolgt, so kann eine Stufe, ein Teil der Stufen oder alle Stufen als Diafiltration ausgestaltet sein. Wird als Lösungsmittel für die Reaktion das Produkt (I) eingesetzt, so wird als zusätzlich zugeführte Flüssigkeit bei der Diafiltration vorzugsweise auch die Verbindung (I) eingesetzt.

In einer weiteren bevorzugten Ausführungsform wird bei der Membrantrennung in Schritt b) die mit dem Permeat abgetrennte Menge Flüssigkeit nicht ergänzt. Eine Ultrafiltration, bei der die abgetrennte Menge an Permeat nicht ergänzt wird, wird erfindungsgemäß als Konzentrierung bezeichnet. Wenn die Auftrennung mittels Membran in Schritt b) in zwei oder mehr als zwei Stufen erfolgt, so kann eine der Stufen, ein Teil der Stufen oder alle Stufen als Konzentrierung ausgestaltet sein.

In einer bevorzugten Ausführungsform umfasst die Membranauftrennung mehrere Stufen, die in Reihe geschaltet sind. Dabei wird der Feedstrom einer ersten Membranauftrennung (erste Stufe) zugeführt, wobei der resultierende Retentatstrom der Folgestufe zurückgeführt wird. Der aus der letzten Stufe entnommene Retentatstrom wird abschließend einer Aufarbeitung zur Gewinnung eines die hochmolekularen Anteile der polymeren Nebenprodukte enthaltenden Ausschleusstroms und eines an Verbindung (I) und/oder Lösungsmittel angereicherten Stroms unterzogen.

In einer speziellen Ausführungsform umfasst die Auftrennung mittels Membran in Schritt b) zuerst wenigstens einen Konzentrierungsschritt und anschließend wenigstens einen Diafiltrationsschritt.

Des Weiteren bevorzugt erfolgt die Auftrennung mittels Membran in Schritt b) kontinuierlich.

Geeignete semipermeable Membranen weisen eine ausreichende Durchlässigkeit für den homogen im Reaktionsmedium vorliegenden Katalysator auf. Sie weisen zudem ein ausreichendes Rückhaltevermögen für den im Reaktionsmedium enthaltenen hochmolekularen Anteil der polymeren Nebenprodukte auf, d. h. sie sind befähigt, höhermolekulare Verbindungen, die sich z. B. durch Oligomerisierung oder Polymerisierung der Oxirane (II) bilden, zurückzuhalten.

Zur Membranauftrennung wird wenigstens eine Membran mit einer Trenngrenze im Bereich von 500 bis 20000 Dalton, bevorzugt 750 bis 10000 Dalton, insbesondere 1000 bis 5000 Dalton, eingesetzt. Die mittlere durchschnittliche Porengröße der Membran beträgt im Allgemeinen 0,8 bis 20 nm, vorzugsweise 0,9 bis 10 nm, besonders bevorzugt 1 bis 5 nm.

Die erfindungsgemäß eingesetzten semipermeablen Membranen weisen wenigstens eine Trennschicht auf, die aus einem oder mehreren Materialien bestehen kann. Diese Materialien sind vorzugsweise ausgewählt unter organischen Polymeren, keramischen Materialien, Metallen, Kohlenstoff und Kombination davon. Geeignete Materialien sind bei der Filtrationstemperatur im Feedmedium stabil. Bevorzugt sind Membranen aus wenigstens einem anorganischen Material.

Geeignete keramische Materialien sind beispielsweise α-Aluminiumoxid, Zirkoniumoxid, Titandioxid, Siliciumcarbid oder gemischte keramische Werkstoffe.

Geeignete organische Polymere sind z. B. Polypropylene, Polytetrafluorethylene, Polyvinylidendifluoride, Polysulfone, Polyethersulfone, Polyetherketone, Polyamide, Polyimide, Polyacrylnitrile, Regeneratcellulose, Siliconpolymere.

Besonders bevorzugt wird in dem erfindungsgemäßen Verfahren eine aus mehreren Schichten aufgebaute anorganische Membran eingesetzt.

Aus mechanischen Gründen sind die Trennschichten in der Regel auf einer ein- oder mehrschichtigen porösen Unterstruktur aus dem gleichen oder auch mehreren unterschiedlichen Materialien wie die Trennschicht aufgebracht. Beispiele für mögliche Materialkombinationen sind in der folgenden Tabelle aufgeführt:

| | |
|---|---|
| Trennschicht | Unterstruktur (gröber als Trennschicht) |
| Metall | Metall |
| Keramik | Metall, Keramik oder Kohlenstoff |
| Polymer | Polymer, Metall, Keramik oder Keramik auf Metall |
| Kohlenstoff | Kohlenstoff, Metall oder Keramik |
| Keramik: z. B. α-Al₂O₃, ZrO₂, TiO₂, SiC, gemischte keramische Werkstoffe | |
| Polymer: z. B. PP, PTFE, PVDF, Polysulfon, Polyethersulfon, Polyetheretherketon, Polyamid, Polyacrylnitril, Regeneratcellulose | |

Besonders bevorzugt sind Trennschichten aus Keramik.

Die Membranen können prinzipiell in Flach-, Rohr-, Multikanalelement-, Kapillar- oder Wickelgeometrie eingesetzt werden, für die entsprechende Druckgehäuse, die eine Trennung zwischen Retentat und dem Permeat erlauben, verfügbar sind.

Die optimalen transmembranen Drücke zwischen Retentat und Permeat sind abhängig von dem Durchmesser der Membranporen, den hydrodynamischen Bedingungen, die den Deckschichtaufbau beeinflussen, und der mechanischen Stabilität der Membran bei der Filtrationstemperatur. Sie liegen im Allgemeinen in einem Bereich von 0,2 bis 30 bar, besonders bevorzugt in einem Bereich von 0,5 bis 20 bar. Höhere transmembrane Drücke führen in der Regel zu höheren Permeatflüssen. Dabei kann in dem Fall, in dem mehrere Module in Serie geschaltet sind, der Transmembrandruck für jeden Modul durch Anhebung des Permeatdruckes abgesenkt und damit angepasst werden. Die Betriebstemperatur ist abhängig von der Membranstabilität und der Temperaturstabilität des Feeds. Ein geeigneter Temperaturbereich für die Membrantrennung in Schritt b) ist 20 bis 90 °C, bevorzugt 40 bis 80 °C. Dabei können die Schmelzpunkte der Produkte den Temperaturbereich einschränken. Höhere Temperaturen führen in der Regel zu höheren Permeatflüssen. Die erreichbaren Permeatflüsse sind stark von der eingesetzten Membranart und Membrangeometrie, von den Prozessbedingungen, von der Feedzusammensetzung (im Wesentlichen der Polymerkonzentration) abhängig. Die Flüsse liegen typischerweise in einem Bereich von 0,5 bis 100 kg/m²/h, bevorzugt 1 bis 50 kg/m²/h.

Folgende Membranen sind z. B. einsetzbar:

| Hersteller | Membran | Trenngrenze (kD) Porendurchmesser (nm) |
|---|---|---|
| Inopor GmbH | γ-Al₂O₃ auf Keramik /1, 2 | 5 nm; 7,5 kD |
| | TiO₂ auf Keramik/1, 2 | 5 nm; 8,5 kD |
| | TiO₂ auf Keramik /1, 2 | 0,9 nm; 0,5 kD |
| | TiO₂ auf Keramik /1, 2 | 1 nm; 0,8 kD |
| | ZrO₂ auf Keramik /1, 2 | 3 nm; 2 kD |
| Atech innovations GmbH | UF / TiO₂ auf α-Al₂O₃ / 1, 2 | 5, 10 und 20 kD |
| Rhodia / Orelis | UF / ZrO₂ oder TiO₂ auf Keramik / 1,2 | 15 kD |
| Pall-Schumacher | UF / TiO₂ oder ZrO₂ auf Keramik / 1,2 | 5und 10 nm |
| Creavis | UF / ZrO₂ auf α-Al₂O₃ und Metall / 3 | 25 nm |
| 1: Rohrmembran; 2: Mehrkanalelement; 3: Flachmembran für Wickel-, Taschen-, Plattenstapel- oder Sondermodule mit bewegter Membran bzw. Rühraggregaten zwischen den Membranen | | |

Die Membrantrennung in Schritt b) kann bei ansonsten kontinuierlicher Reaktionsführung auch diskontinuierlich erfolgen, beispielsweise durch mehrmaligen Durchgang durch die Membranmodule. Vorzugsweise erfolgt die Membrantrennung in Schritt b) kontinuierlich, beispielsweise durch einmaligen Durchgang durch eine oder mehrere nacheinander geschaltete Membran-Trennstufen.

Um auf der Membranoberfläche einen nennenswerten Aufbau einer Deckschicht aus zurückgehaltenen hochmolekularen Anteil der polymeren Nebenprodukte zu vermeiden, der zu einer Abnahme des Permeatflusses führen kann, hat sich Umpumpen, mechanische Bewegung der Membran oder der Einsatz von Rühraggregaten zwischen den Membranen als nützlich erwiesen. Diese Maßnahmen dienen zur Erzeugung einer Relativgeschwindigkeit zwischen Membran und zu trennendem Reaktionsaustrag in einem Bereich von 0,1 bis 10 m/s.

Aus dem Retentat können die hochsiedenden Verunreinigungen durch an sich bekannte Verfahren abgetrennt werden. Vorzugsweise wird das Retentat einer Destillation unter Erhalt eines an hochsiedenden Verbindungen angereicherten Ausschleusstroms und eins an Verbindung (I) angereicherten Stroms unterzogen. Die Destillation kann mit an sich bekannten Apparaturen erfolgen, z. B. durch Einsatz wenigstens eines Kurzweg-Verdampfers.

Die Erfindung wird im Folgenden anhand von Figuren erläutert, die bevorzugte Ausführungen des erfindungsgemäßen Verfahrens darstellen, ohne die Erfindung darauf zu beschränken.
- Fig. 1: zeigt eine schematische Darstellung einer zur Durchführung des erfindungsgemäßen Verfahrens geeigneten Anlage.
- Fig. 2: zeigt eine schematische Darstellung einer kontinuierlich betriebenen zweistufigen Membrankaskade.
- Fig. 3: zeigt eine schematische Darstellung der in den Beispielen verwendeten Apparatur.

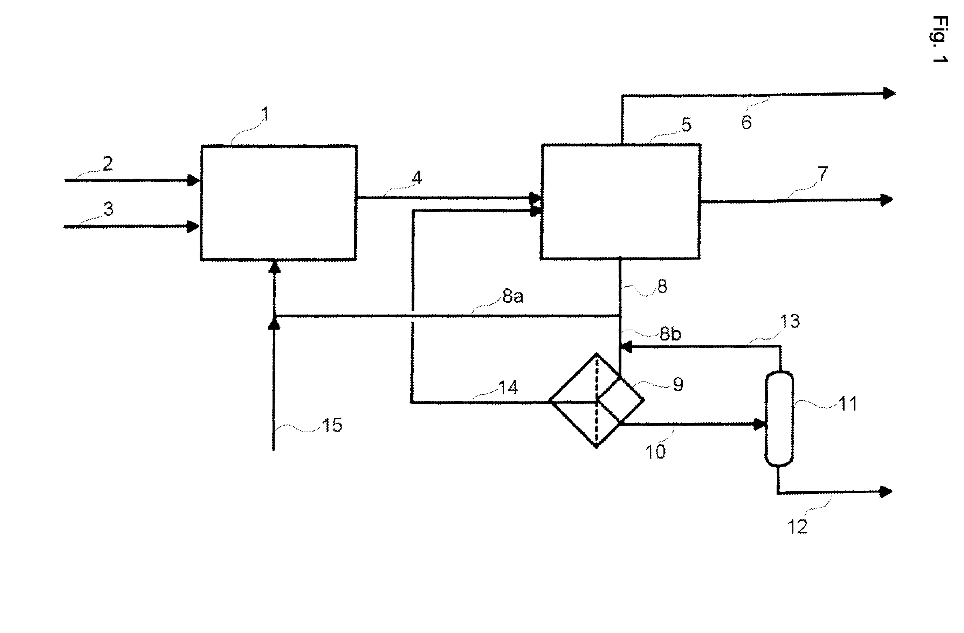

Fig. 1 stellt ein Schema einer zur Durchführung des erfindungsgemäßen Verfahrens geeigneten Anlage dar, wobei aus Gründen der Übersichtlichkeit auf die Wiedergabe solcher Details verzichtet wurde, die für die Erläuterung der Erfindung nicht relevant sind. Die Anlage umfasst eine Reaktionszone (1), die wenigstens einen Reaktor umfasst. Über die Rohrleitung (2) wird ein Oxiran (wie Ethylenoxid) in die Reaktionszone (1) eingeleitet und über die Rohrleitung (3) wird CO₂ in die Reaktionszone (1) eingeleitet. Über die von der Reaktionszone (1) abgehende Rohrleitung wird ein Austrag (4) aus dem Reaktor (1) entnommen und der Aufarbeitungsstufe (5) zugeführt. In einer speziellen Ausführungsform wird der Austrag (4) zunächst einem Entspannungsgefäß (nicht abgebildet) zugeführt, wobei eine Phasentrennung in eine kohlendioxidhaltige Gasphase und eine Flüssigphase erfolgt, die anschließend einer weiteren Aufarbeitung in der Aufarbeitungsstufe (5) zugeführt wird. Die bei der Entspannung erhaltene Gasphase kann zusätzlich Anteile nicht umgesetzten Oxirans enthalten. In der Aufarbeitungsstufe (5) erfolgt eine destillative Auftrennung unter Erhalt einer Gasphase (6), die aus den niedrigsiedenden Komponenten des Reaktionsaustrags (d. h. im Wesentlichen aus Kohlendioxid und/oder Oxiran) besteht, einen im Wesentlichen aus dem 1,3-Dioxolan-2-on (wie Ethylencarbonat) bestehenden Strom (7) und einem Sumpfprodukt (8), welches im Wesentlichen aus dem 1,3-Dioxolan-2-on, dem Katalysator und den polymeren Nebenprodukten der Reaktion besteht. Der den Katalysator und die polymeren Nebenprodukte enthaltene Strom (8) wird geteilt in einen ersten Teilstrom (8a), der in die Reaktionszone (1) zurückgeführt wird, und einen zweiten Teilstrom (8b), der der Membrantrennung (9) zugeführt wird. Die Membrantrennung (9) kann dabei ein- oder mehrstufig ausgeführt sein. In der Membrantrennung (9) wird ein Retentatstrom (10) erhalten, der die von der semipermeablen Membran zurückgehaltenen Hochsiederanteile des Reaktoraustrags sowie Oxiran und gegebenenfalls geringe Anteile von nicht abgetrenntem Katalysator enthält. Dieser Retentatstrom (10) wird einer Aufarbeitungsstufe (11) zugeführt, die in einer speziellen Ausführungsform als Kurzweg-Verdampfer ausgeführt ist. Der in der Aufarbeitungsstufe (11) erhaltene Hochsiederstrom (12) wird aus dem Verfahren ausgeschleust. Der ebenfalls erhaltene an 1,3-Dioxolan-2-on angereicherte Strom (13) wird erneut der Membrantrennung (9) zugeführt. Das bei der Membrantrennung (9) erhaltene Permeat (14), das im Wesentlichen aus dem 1,3-Dioxolan-2-on, dem Katalysator sowie den Anteilen an Hochsiedern besteht, die in der Membrantrennung (9) nicht zurückgehalten wurden, wird in einer ersten Ausführungsform in die Aufarbeitungsstufe (5) zurückgeführt. In einer zweiten (nicht abgebildeten) Ausführungsform wird der Permeatstrom (14) in die Reaktionszone (1) zurückgeführt. Über den Zuführstrom (15) kann im Bedarfsfall frischer Katalysator in die Reaktionszone (1) eingespeist werden.

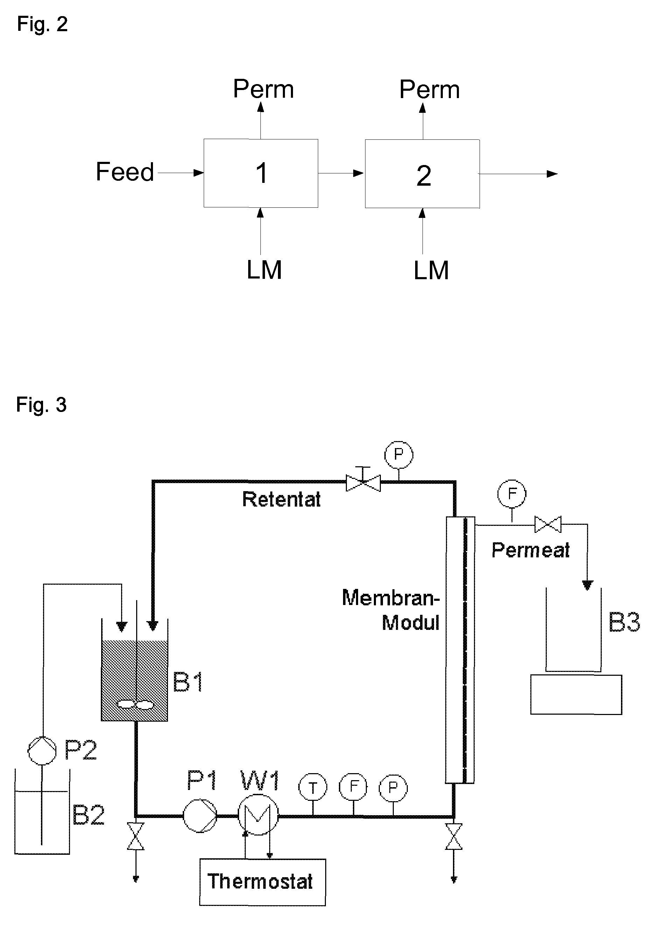

Fig. 2 zeigt schematisch eine bevorzugte Ausgestaltung der Auftrennung eines katalysator- und hochsiederhaltigen Stroms mittels einer kontinuierlich betriebenen zweistufigen Membrankaskade, wobei die zwei Stufen als Konzentrierungsschritt (Lösungsmittelstrom = 0) oder Diafiltrationsschritt (Lösungsmittelstrom = Permeatstrom) oder als Mischform aus Konzentrierungs- und Diafiltrationsschritt (0 < Lösungsmittelstrom < Permeatstrom) ausgestaltet sein können.

Für kontinuierlich betriebene Membranstufen bezogen auf eine Stufe sind hierbei:

| | |
|---|---|
| LM | = Lösungsmittel |
| Perm | = Permeat |
| Diafiltration: | LM-Strom = Perm-Strom |
| Konzentrierung: | LM-Strom = 0 |
| | |
| Mischform aus Diafiltration und Konzentrierung: | 0 < LM-Strom < Perm-Strom |

Die Definitionen für Konzentrierung und Diafiltration unterscheiden sich dabei für die abgebildete kontinuierliche Fahrweise und die ebenfall geeignete Batch-Fahrweise:

### kontinuierliche Fahrweise:

| | |
|---|---|
| Diafiltration: | LM-Strom = Perm-Strom |
| Mischform: | 0 < LM-Strom < Perm-Strom |
| Konzentrierung: | LM-Strom = 0 |

### Batch-Fahrweise:

| | |
|---|---|
| Diafiltration: | LM-Strom = Perm-Strom |
| Konzentrierung: | LM-Strom = 0 |

Figur 3 zeigt schematisch die in den Beispielen eingesetzte und in Batch-Fahrweise betriebene Apparatur.
B1 = Kreislaufbehälter
B2 = Vorlage für Diafiltriermedium
B3 = Permeat-Auffangbehälter (zur Permeatfluss-Messung auf Waage)
P1 = Kreislaufpumpe
P2 = Dosierpumpe
W1 = Wärmetauscher
P = Druckmessung
T = Temperaturmessung
F = Durchflussmessung

Die Erfindung wird anhand der folgenden, nicht einschränkenden Beispiele näher erläutert.

Die Versuche zur Abtrennung des hochmolekularen Anteils der polymeren Nebenprodukte vom Katalysator sowie dem niedermolekularen Anteil der polymeren Nebenprodukte erfolgten mit einer keramischen Mehrschichtmembran der Firma Inopor GmbH, deren Zirkondioxid-Trennschicht einen mittleren Porendurchmesser von 3 nm und eine molekulare Trenngrenze von 2 kD aufwies. Eingesetzt wurde ein 19/3,5 Mehrkanalelement (in einem Element befinden sich 19 Bohrungen mit einem Innendurchmesser von 3,5 mm, auf deren Innenseite die Membran aufgebracht ist) mit einer Länge von 50 cm und einer Fläche von 0,098 m². Eingesetzt wurden katalysatorhaltige Reaktorausträge aus der Ethylencarbonatsynthese (Katalysator: Bromidsalzgemisch), die destillativ von Niedersiedern befreit und aus denen Ethylencarbonat teilweise destillativ abgetrennt war.

Dieser ethylencarbonat-, katalysator- und polymerhaltige Einsatz wurde in allen Versuchen batchweise aufgearbeitet. Dazu wurde das eingesetzte Material aus einem Kreislaufgefäß mit einer Pumpe auf einen Druck von 15 bar gebracht und mit einer Temperatur von 70 °C und einer Geschwindigkeit von 2 m/s durch die Membranrohre geführt, danach wieder auf Normaldruck entspannt und zurück ins Kreislaufgefäß geleitet. Bei Normaldruck abgetrenntes Permeat wurde zur Permeatfluss-Bestimmung in einem Gefäß auf einer Waage aufgefangen und kontinuierlich durch eine gleich große Menge an Diafiltriermedium (in allen Versuchen Ethylencarbonat) ersetzt. Die Diafiltration erfolgte in der Regel mit einem Lösungsmittelaustauschkoeffizient MA von ca. 3. D. h. bei einem Einsatz von x kg wurden 3x kg Permeat abgezogen und 3x kg Ethylencarbonat so zum Retentat zudosiert, dass die Retentatmenge konstant blieb. Am Ende der Versuche wurden die Einsätze (Feed) und die Austräge (Retentat) analysiert. Für die Katalysatoranalytik wurde das gemeinsame Anion des Katalysator-Salzgemisches (Bromid) verwendet.

| Versuch | mittlerer Permeat-Fuss (kg/m²/h) | MA | Feed | | | Retentat | | Ausbeute bz. Retentat | |
|---|---|---|---|---|---|---|---|---|---|
| | | | M_{W} HS (kg/mol) | Konzentration | | Konzentration | | | |
| | | | | Br (%) | HS (%) | Br (%) | HS (%) | Br⁻ (%) | HS (%) |
| | | | | | | | | | |
| 1 | 8,6 | 3,01 | 10 | 1,215 | 23,2 | 0,079 | 14,3 | 6,5 | 62 |
| 2 | 13,4 | 2,93 | 7 | 1,438 | 14,1 | 0,184 | 7,0 | 12,8 | 50 |

| | | | | | | | | | |
|---|---|---|---|---|---|---|---|---|---|
| M_{W} : gewichtsgemitteltes Molekulargewicht (GPC) HS : Hochsieder Br : Bromid (potentiometrische Titration) | | | | | | | | | |

Die Versuche zeigen, dass aus dem katalysator- und polymerhaltigen Einsatz der Katalysator und der niedermolekulare Anteil der polymeren Nebenprodukte abgereichert werden können und dass der höhermolekulare Anteil der polymeren Nebenprodukte durch die Membran zurückgehalten wird. Damit ist es möglich einen katalysatorarmen, polymer- und ethylencarbonathaltigen Strom zur Ausschleusung des höhermolekularen Anteils der polymeren Nebenprodukte bereitzustellen.

## Patentansprüche

1. Verfahren zur kontinuierlichen Herstellung eines 1,3-Dioxolan-2-ons der allgemeinen Formel I 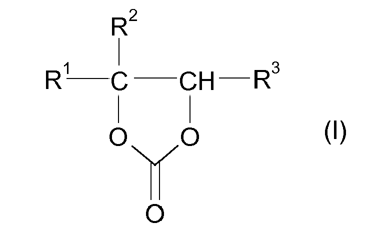 worin
R¹ für Wasserstoff oder einen organischen Rest mit 1 bis 40 Kohlenstoffatomen steht,
R² und R³ unabhängig voneinander für Wasserstoff oder C₁-C₄-Alkyl stehen, wobei R² und R³ auch miteinander zu einem fünf- oder sechsgliedrigen Ring verbunden sein können, bei dem man:
a) in einer Reaktionszone ein Oxiran der allgemeinen Formel II 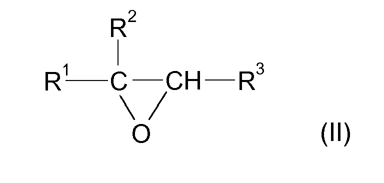 worin R¹, R² und R³ die zuvor angegebenen Bedeutungen besitzen, mit Kohlendioxid in flüssiger Phase in Gegenwart eines in der flüssigen Phase homogen vorliegenden Katalysators umsetzt,
b) aus der Reaktionszone einen flüssigen Austrag entnimmt, der polymere Nebenprodukte der Umsetzung enthält, und einer Aufarbeitung unterzieht, die eine Auftrennung mittels einer semipermeablen Membran unter Erhalt eines Permeats und eines Retentats umfasst, wobei ein hochmolekularer Anteil der polymeren Nebenprodukte von der Membran zurückgehalten wird, wobei in Schritt b) vom Austrag aus der Reaktionszone ein im Wesentlichen aus der Verbindung (I), dem Katalysator und den polymeren Nebenprodukten bestehenden Strom abgetrennt und zumindest teilweise der Auftrennung mittels einer semipermeablen Membran unterzogen wird, wobei der Katalysator zumindest teilweise in das Permeat übergeht, wobei der Permeat wenigstens 70 % des in dem zur Membrantrennung eingesetzten Strom enthaltenen Katalysators enthält, und
c) aus dem Retentat einen den hochmolekularen Anteil der polymeren Nebenprodukte enthaltenden Ausschleusstrom bereitstellt.

2. Verfahren nach Anspruch 1, bei dem in Schritt b) ein niedermolekularer Anteil der Nebenprodukte in das Permeat übergeht.

3. Verfahren nach Anspruch 1, bei dem man zusätzlich:
d) das Permeat zumindest teilweise in die Reaktionszone in Schritt a) und/oder die Aufarbeitung in Schritt b) zurückführt.

4. Verfahren nach Anspruch 1, bei dem man den vom Austrag aus der Reaktionszone abgetrennten, im Wesentlichen aus der Verbindung (I), dem Katalysator und den polymeren Nebenprodukten bestehenden Strom teilweise in die Reaktionszone zurückführt.

5. Verfahren nach Anspruch 1, bei dem man den im Wesentlichen aus der Verbindung (I), dem Katalysator und den polymeren Nebenprodukten bestehenden Strom dadurch erhält, dass man vom Austrag aus der Reaktionszone einen im Wesentlichen aus nicht ungesetztem Kohlendioxid und/oder dem Oxiran der Formel (II) bestehenden gasförmigen Strom und einen im Wesentlichen aus der Verbindung (I) bestehenden Strom abtrennt.

6. Verfahren nach Anspruch 5, bei dem man den Austrag aus der Reaktionszone zuerst einem Entspannungsschritt unterzieht, wobei eine Auftrennung in eine Flüssigphase, die im Wesentlichen aus der Verbindung (I), polymeren Nebenprodukten, dem homogen gelösten Katalysator und gegebenenfalls geringen Mengen an gelöstem Kohlendioxid und/oder Oxiran (II) besteht, und eine Gasphase, die im Wesentlichen aus Kohlendioxid und/oder Oxiran (II) besteht, erfolgt, und die Flüssigphase einer Destillation unter Erhalt eines im Wesentlichen aus der Verbindung (I) bestehenden Stroms und eines im Wesentlichen aus der Verbindung (I), dem Katalysator und den polymeren Nebenprodukten bestehenden Stroms unterzieht.

7. Verfahren nach Anspruch 5, bei dem man den Austrag aus der Reaktionszone einer destillativen Auftrennung in
- einen im Wesentlichen aus nicht umgesetztem Kohlendioxid und/oder Oxiran (II) bestehenden Strom,
- einen im Wesentlichen aus der Verbindung (I) bestehenden Strom, und
- einen im Wesentlichen aus der Verbindung (I), dem Katalysator und den polymeren Nebenprodukten bestehenden Strom unterzieht.

8. Verfahren nach einem der vorhergehenden Ansprüche, wobei die Auftrennung mittels Membran in Schritt b) in zwei oder mehr Stufen erfolgt.

9. Verfahren nach Anspruch 8, wobei die Auftrennung in Schritt b) wenigstens einen Diafiltrationsschritt umfasst, bei dem die mit dem Permeat abgetrennte Menge Flüssigkeit retentatseitig zumindest teilweise durch zusätzlich zugeführte Flüssigkeit ergänzt wird.

10. Verfahren nach Anspruch 8, wobei die Auftrennung in Schritt b) wenigstens einen Konzentrierungsschritt umfasst, bei dem die mit dem Permeat abgetrennte Menge Flüssigkeit nicht durch zusätzlich zugeführte Flüssigkeit ergänzt wird.

11. Verfahren nach Anspruch 8, wobei die Auftrennung in Schritt b) eine Mischform aus einem Diafiltrationsschritt und einem Konzentrierungsschritt umfasst, bei dem die mit dem Permeat abgetrennte Menge Flüssigkeit teilweise durch zusätzlich zugeführte Flüssigkeit ergänzt wird.

12. Verfahren nach einem der vorhergehenden Ansprüche, bei dem die Auftrennung mittels Membran in Schritt b) kontinuierlich durchgeführt wird.

13. Verfahren nach einem der vorhergehenden Ansprüche, bei dem zur Membranauftrennung wenigstens eine Membran mit einer Trenngrenze im Bereich von 500 bis 20000 Dalton, vorzugsweise 750 bis 10000 Dalton, besonders bevorzugt 1000 bis 5000 Dalton eingesetzt wird.

14. Verfahren nach einem der vorhergehenden Ansprüche, bei dem zur Membranauftrennung wenigstens eine Membran mit einem Porendurchmesser im Bereich von 0,8 bis 20 nm, vorzugsweise 0,9 bis 10 nm, besonders bevorzugt 1 bis 5 nm eingesetzt wird.

15. Verfahren nach einem der vorhergehenden Ansprüche zur Herstellung von Ethylencarbonat oder Propylencarbonat.

## Claims

1. A process for the continuous preparation of a 1,3-dioxolan-2-one of the general formula I 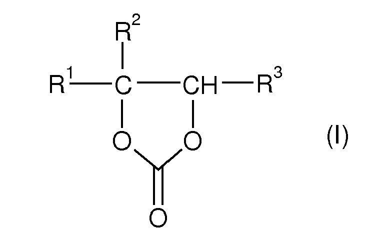 where
R¹ is hydrogen or an organic radical having from 1 to 40 carbon atoms,
R² and R³ are each, independently of one another, hydrogen or C₁-C₄-alkyl, where R² and R³ may also be joined to one another to form a five- or six-membered ring, wherein:
a) an oxirane of the general formula II 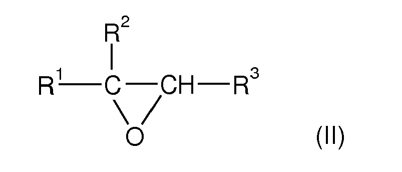 where R¹, R² and R³ are as defined above, is reacted with carbon dioxide in the liquid phase in the presence of a catalyst dissolved homogeneously in the liquid phase in a reaction zone,
b) a liquid discharge comprising polymeric by-products of the reaction is taken from the reaction zone and subjected to a work-up comprising fractionation by means of a semipermeable membrane to give a permeate and a retentate, with a high molecular weight fraction of the polymeric by-products being retained by the membrane, a stream consisting essentially of the compound (I), the catalyst and the polymeric by-products being separated off from the discharge from the reaction zone in step b) and being subjected at least partly to the fractionation by means of a semipermeable membrane, with the catalyst passing at least partly into the permeate, with the permeate comprising at least 70% of the catalyst comprised in the stream used for the membrane separation, and
c) a purge stream comprising the high molecular weight fraction of the polymeric by-products is provided from the retentate.

2. The process according to claim 1, wherein a low molecular weight fraction of the by-products passes into the permeate in step b).

3. The process according to claim 1, wherein, in addition:
d) the permeate is recirculated at least partly to the reaction zone in step a) and/or the work-up in step b).

4. The process according to claim 1, wherein the stream consisting essentially of the compound (I), the catalyst and the polymeric by-products which has been separated off from the discharge from the reaction zone is at least partly recirculated to the reaction zone.

5. The process according to claim 1, wherein the stream consisting essentially of the compound (I), the catalyst and the polymeric by-products is obtained by separating off a gaseous stream consisting essentially of unreacted carbon dioxide and/or the oxirane of the formula (II) and a stream consisting essentially of the compound (I) from the discharge from the reaction zone.

6. The process according to claim 5, wherein the discharge from the reaction zone is firstly subjected to a depressurization step in which fractionation into a liquid phase consisting essentially of the compound (I), polymeric by-products, the homogeneously dissolved catalyst and optionally small amounts of dissolved carbon dioxide and/or oxirane (II) and a gas phase consisting essentially of carbon dioxide and/or oxirane (II) occurs and the liquid phase is subjected to a distillation to give a stream consisting essentially of the compound (I) and a stream consisting essentially of the compound (I), the catalyst and the polymeric by-products.

7. The process according to claim 5, wherein the discharge from the reaction zone is subjected to a fractional distillation into
- a stream consisting essentially of unreacted carbon dioxide and/or oxirane (II),
- a stream consisting essentially of the compound (I) and
- a stream consisting essentially of the compound (I), the catalyst and the polymeric by-products.

8. The process according to any of the preceding claims, wherein the fractionation by means of a membrane in step b) is carried out in two or more stages.

9. The process according to claim 8, wherein the fractionation in step b) comprises at least one diafiltration step in which the amount of liquid separated off with the permeate is at least partly replaced on the retentate side by additionally introduced liquid.

10. The process according to claim 8, wherein the fractionation in step b) comprises at least one concentration step in which the liquid separated off with the permeate is not replaced by additionally introduced liquid.

11. The process according to claim 8, wherein the fractionation in step b) comprises a mixed form comprising a diafiltration step and a concentration step in which the amount of liquid separated off with the permeate is partly replaced by additionally introduced liquid.

12. The process according to any of the preceding claims, wherein the fractionation by means of a membrane in step b) is carried out continuously.

13. The process according to any of the preceding claims, wherein at least one membrane having a separation limit in the range from 500 to 20 000 dalton, preferably from 750 to 10 000 dalton, particularly preferably from 1000 to 5000 dalton, is used for the membrane fractionation.

14. The process according to any of the preceding claims, wherein at least one membrane having a pore diameter in the range from 0.8 to 20 nm, preferably from 0.9 to 10 nm, particularly preferably from 1 to 5 nm, is used for the membrane fractionation.

15. The process according to any of the preceding claims for preparing ethylene carbonate or propylene carbonate.

## Revendications

1. Procédé de préparation continue d'une 1,3-dioxolann-2-one de formule générale I 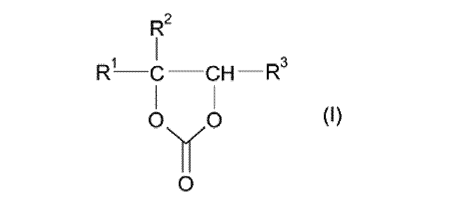 dans laquelle
R¹ représente un atome d'hydrogène ou un radical organique ayant 1 à 40 atomes de carbone,
R² et R³ représentent chacun indépendamment de l'autre un atome d'hydrogène ou un groupe alkyle en C₁-C₄, R² et R³ pouvant aussi être reliés l'un à l'autre pour former un noyau à cinq ou six chaînons, dans lequel :
a) dans une zone de réaction, on fait réagir un oxiranne de formule générale II 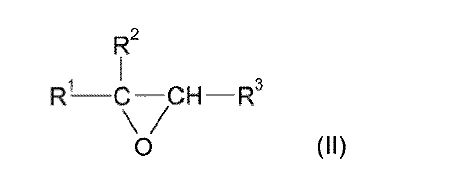 dans laquelle R¹, R² et R³ ont les significations données ci-dessus, avec du dioxyde de carbone en phase liquide en présence d'un catalyseur présent sous forme homogène dans la phase liquide,
b) on prélève de la zone de réaction un produit liquide, qui contient des sous-produits polymères de la réaction, et on le soumet à un traitement qui comprend une séparation à l'aide d'un membrane semi-perméable pour obtenir un perméat et un rétentat, une partie à grande masse moléculaire des sous-produits polymères étant retenue par la membrane ; dans l'étape b), un courant constitué pour l'essentiel du composé (I), du catalyseur et des sous-produits polymères, étant séparé du produit de la zone de réaction, et étant au moins partiellement soumis à la séparation à l'aide d'une membrane semi-perméable, le perméat contenant au moins 70 % du catalyseur contenu dans le courant utilisé pour la séparation sur membrane, et
c) à partir du rétentat, on prépare un courant de décharge contenant la partie à grande masse moléculaire des sous-produits polymères.

2. Procédé selon la revendication 1, dans lequel, dans l'étape b), une partie à faible masse moléculaire des sous-produits passe dans le perméat.

3. Procédé selon la revendication 1, dans lequel, en outre :
d) on renvoie le perméat au moins partiellement dans la zone de réaction de l'étape a) et/ou au traitement de l'étape b).

4. Procédé selon la revendication 1, dans lequel on renvoie partiellement dans la zone de réaction le courant séparé du produit de la zone de réaction, constitué pour l'essentiel du composé (I), du catalyseur et des sous-produits polymères.

5. Procédé selon la revendication 1, dans lequel on obtient le courant constitué pour l'essentiel du composé (I), du catalyseur et des sous-produits polymères, par le fait que l'on sépare du produit de la zone de réaction un courant gazeux constitué pour l'essentiel de dioxyde de carbone n'ayant pas réagi et/ou de l'oxiranne de formule (II), et un courant constitué pour l'essentiel du composé (I).

6. Procédé selon la revendication 5, dans lequel on soumet le produit de la zone de réaction d'abord à une étape de détente, une séparation ayant lieu en une phase liquide qui pour l'essentiel est constituée du composé (I), des sous-produits polymères, du catalyseur dissous d'une manière homogène et éventuellement de faibles quantités de dioxyde de carbone dissous et/ou de l'oxiranne (II), et une phase gazeuse, qui pour l'essentiel est constituée de dioxyde de carbone et/ou de l'oxiranne (II), et la phase liquide étant soumise à une distillation, avec obtention d'un courant constitué pour l'essentiel du composé (I) et d'un courant pour l'essentiel constitué du composé (I), du catalyseur et des sous-produits polymères.

7. Procédé selon la revendication 5, dans lequel on soumet le produit de la zone de réaction à une séparation par distillation
- en un courant pour l'essentiel constitué de dioxyde de carbone n'ayant pas réagi et/ou de l'oxiranne (II),
- en un courant pour l'essentiel constitué du composé (I), et
- en un courant pour l'essentiel constitué du composé (I), du catalyseur et des sous-produits polymères.

8. Procédé selon l'une des revendications précédentes, dans lequel la séparation à l'aide de la membrane de l'étape b) a lieu en deux étapes ou plus.

9. Procédé selon la revendication 8, dans lequel la séparation de l'étape b) comprend au moins une étape de diafiltration, dans laquelle la quantité de liquide séparée avec le perméat est, côté rétentat, au moins partiellement complétée par un liquide amené en plus.

10. Procédé selon la revendication 8, dans lequel la séparation de l'étape b) comprend au moins une étape de concentration, dans laquelle la quantité de liquide séparée avec le perméat n'est pas complétée par du liquide amené en plus.

11. Procédé selon la revendication 8, dans lequel la séparation de l'étape b) comprend une forme mixte constituée d'une étape de diafiltration et d'une étape de concentration, dans laquelle la quantité de liquide séparée avec le perméat est partiellement complétée par du liquide amené en plus.

12. Procédé selon l'une des revendications précédentes, dans lequel la séparation à l'aide de la membrane de l'étape b) est réalisée en continu.

13. Procédé selon l'une des revendications précédentes, dans lequel on utilise pour la séparation sur membrane au moins une membrane ayant une limite de séparation comprise dans la plage de 500 à 20 000 Daltons, de préférence de 750 à 10 000 Daltons, d'une manière particulièrement préférée de 1000 à 5000 Daltons.

14. Procédé selon l'une des revendications précédentes, dans lequel on utilise pour la séparation sur membrane au moins une membrane ayant un diamètre de pore compris dans la plage de 0,8 à 20 nm, de préférence de 0,9 à 10 nm, d'une manière particulièrement préférée de 1 à 5 nm.

15. Procédé selon l'une des revendications précédentes pour la préparation de carbonate d'éthylène ou de carbonate de propylène.
